(19) Europäisches Patentamt
European Patent Office
Office européen des brevets

(11) EP 2 490 659 B1

(12) EUROPÄISCHE PATENTSCHRIFT

(45) Veröffentlichungstag und Bekanntmachung des Hinweises auf die Patenterteilung:
**01.03.2017 Patentblatt 2017/09**

(21) Anmeldenummer: **10776320.3**

(22) Anmeldetag: **21.10.2010**

(51) Int Cl.:
***A61K 8/73*** *(2006.01)* ***A61Q 5/06*** *(2006.01)*
***A61K 8/81*** *(2006.01)*

(86) Internationale Anmeldenummer:
**PCT/EP2010/065860**

(87) Internationale Veröffentlichungsnummer:
**WO 2011/048177 (28.04.2011 Gazette 2011/17)**

# (54) MITTEL ZUR TEMPORÄREN VERFORMUNG KERATINHALTIGER FASERN, ENTHALTEND EINE NICHTIONISCHE, MITTELS PROPYLENOXID MODIFIZIERTE STÄRKE UND EIN CHITOSAN

COMPOSITION FOR THE TEMPORARY SHAPING OF KERATINIC FIBRES COMPRISING A NONIONIC PROPYLENEOXIDE-MODIFIED STARCH AND A CHITOSAN

PRODUIT POUR LE MAINTIEN DE LA CHEVELURE COMPRENANT UN AMIDON NON IONIQUE MODIFIÉ PAR DE L'OXYDE DE PROPRYLÈNE ET UN CHITOSAN

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.10.2009 DE 102009045925**
**22.10.2009 DE 102009045933**

(43) Veröffentlichungstag der Anmeldung:
**29.08.2012 Patentblatt 2012/35**

(73) Patentinhaber: **Henkel AG & Co. KGaA**
**40589 Düsseldorf (DE)**

(72) Erfinder:
- **MÜLLER, Burkhard**
  **21075 Hamburg (DE)**
- **KAFTAN, Pamela**
  **22525 Hamburg (DE)**
- **BAYERSDÖRFER, Rolf**
  **22589 Hamburg (DE)**
- **SCHWEINSBERG, Matthias**
  **22769 Hamburg (DE)**
- **RÖNISCH, Ralf**
  **42349 Wuppertal (DE)**
- **SCHRIEFERS, Mathias**
  **41239 Mönchengladbach (DE)**
- **DOGAN, Carine**
  **F-91270 Vigneux sur Seine (FR)**
- **KNAPPE, Thorsten**
  **22869 Schenefeld (DE)**

(56) Entgegenhaltungen:
**EP-A1- 0 290 741** **EP-A1- 0 580 514**
**EP-A1- 0 659 394** **EP-A2- 0 097 229**
**EP-A2- 0 948 958** **EP-A2- 0 948 959**
**EP-A2- 0 948 960** **EP-A2- 1 317 916**
**DE-A1- 3 541 305**

- **T.E. Gottschalck, J.E. Bailey: "International Cosmetic Ingredient Dictionary and Handbook", 2008, The Cosmetic, Toiletry and Fragrance Association, Washington, D.C., XP002627782, ISBN: 1-882621-43-3 Bd. 3, Seiten 3187-3215, Seite 3187 - Seite 3192 Seite 3214 - Seite 3215**

Bemerkungen:
Die Akte enthält technische Angaben, die nach dem Eingang der Anmeldung eingereicht wurden und die nicht in dieser Patentschrift enthalten sind.

EP 2 490 659 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft kosmetische Mittel zur temporären Umformung keratinischer Fasern, enthaltend in einem kosmetischen Träger eine Kombination aus mindestens einer Chitosanverbindung und mindestens einer nichtionischen, mittels Propylenoxid modifizierten Stärke.

Stylingmittel zur Verformung keratinischer Fasern sind lange bekannt und finden in verschiedener Ausgestaltung Einsatz zum Aufbau, zur Auffrischung und zur Fixierung von Frisuren, die sich bei vielen Haartypen nur unter Verwendung festigender Wirkstoffe erhalten lassen. Dabei spielen sowohl Haarbehandlungsmittel, die einer permanenten, als auch solche, die einer temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Haargele und Haarwachse werden hingegen in der Regel nicht direkt auf das Haar appliziert, sondern mittels eines Kamms oder der Hände im Haar verteilt.

Die in Mitteln zur temporären Formgebung üblicherweise eingesetzten synthetischen Polymere werden aus entsprechenden synthetisch zugänglichen Monomeren hergestellt. Besagte Monomere werden aus fossilen Stoffen wie beispielsweise Erdöl durch Umwandlung zu den entsprechenden Polymerbausteinen u.a. unter Aufwand von Energie gewonnen.
Im Rahmen eines nachhaltigeren Umganges mit der Natur als Lebensraum und mit den Resourcen bleibt es wünschenswert, für kosmetische Produkte nur solche kosmetische Rohstoffe zu verwenden, die unter möglichst wenig Einsatz von Energie, d.h. aus so genannten nachwachsenden Rohstoffen, zugänglich sind. Eine Mengenreduktion oder gar ein Austausch besagter synthetischer Polymere kann allerdings nur dann vorgenommen werden, wenn die Ersatzpolymere die für den Anwendungszweck gewünschten Eigenschaften aufweisen und den keratinhaltigen Fasern einen ausreichenden, stabilen Halt der aufgeprägten Form (z.B. Frisur) geben.

Des weiteren sollen die Ersatzpolymere auf natürlicher Basis dem Faserkollektiv Volumen verleihen und die Sprungkraft sowie die Geschmeidigkeit der in der Form fixierten, keratinhaltigen Fasern erhalten. Die Bildung von mit dem bloßen Auge sichtbaren Polymerpartikeln auf den keratinhaltigen Fasern muss vermieden werden. Ferner darf die keratinhaltige Faser nicht stumpf wirken, sondern soll natürlich glänzen.

Aufgabe der vorliegenden Erfindung war es daher, eine kosmetische Zusammensetzung bereitzustellen, die eine sehr starke und lang anhaltende Formfixierung bewirkt, eine hohe Volumenwirkung hat, eine gute Faserpflege liefert und die vorgenannten Nachteile nicht aufweist. Dabei soll überwiegend bis möglichst ganz auf den Einsatz der auf fossilen Rohstoffen basierenden, synthetischen Polymere verzichtet werden können.

Ein erster Gegenstand der Erfindung ist daher ein kosmetisches Mittel zur temporären Umformung keratinischer gemäss Anspruch 1

Unter keratinischen Fasern sind dabei erfindungsgemäß Pelze, Wolle, Federn und insbesondere menschliche Haare zu verstehen.

Chitosane stellen Biopolymere dar und werden zur Gruppe der Hydrokolloide gezählt. Chemisch betrachtet, handelt es sich um partiell deacetylierte Chitine unterschiedlichen Molekulargewichtes. Zur Herstellung der Chitosane geht man von Chitin, vorzugsweise den Schalenresten von Krustentieren aus, die als billige Rohstoffe in großen Mengen zur Verfügung stehen. Das Chitin wird dabei üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, die ein mittleres Molekulargewicht (Gewichtsmittel) von 800.000 bis 1.200.000 Dalton, eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% aufweisen.

Erfindungsgemässe Mittel sind dadurch gekennzeichnet, daß sie als Chitosan mindestens ein Neutralisationsprodukt von Chitosan mit mindestens einer organischen Carbonsäure, wie insbesondere Ameisensäure, Essigsäure, Zitronensäure, Milchsäure, Pyrrolidoncarbonsäure, Weinsäure, Glycolsäure, Nicotinsäure, Hydroxyisobuttersäure, Hydroxyisovaleriansäure oder Gemischen dieser Säuren, enthalten. Hierbei ist es erfindungsgemäß bevorzugt, die organische Carbonsäure unter Milchsäure, Ameisensäure, Pyrrolidoncarbonsäure, Nicotinsäure, Hydroxyisobuttersäure, Hydroxyisovaleriansäure oder Gemischen dieser Säuren auszuwählen. Dieses Neutralisationsprodukt kann beispielsweise in einem wässrigen Medium durch Zugabe von Chitosan und der entsprechenden organischen Carbonsäure hergestellt werden.

Geeignete Chitosane sind beispielsweise unter den Handelsbezeichnungen Hydagen® CMF (1 Gew.-% Aktivsubstanz in wässriger Lösung mit 0.4 Gew.-% Glykolsäure, Molekulargewicht 500000 bis 5000000 g/mol Cognis), Hydagen® HCMF (Chitosan (zu 80 % deacetyliert), Molekulargewicht 50000 bis 1000000 g/mol, Cognis), Kytamer® PC (80 Gew.-% Aktivsubstanz an Chitosan pyrolidoncarboxylat (INCI-Bezeichnung: Chitosan PCA), Amerchol) und Chitolam® NB/101 im Handel frei verfügbar.

Die Chitosane sind in den erfindungsgemäßen Mitteln bevorzugt in einer Menge von 0,01 Gew.-% bis 5 Gew.-%, besonders bevorzugt von 0,05 Gew.-% bis 2,0 Gew.-%, ganz besonders bevorzugt von 0,1 Gew.-% bis 1 Gew.-%, jeweils bezogen auf das Gewicht des erfindungsgemäßen Mittels, enthalten.

Stärke ist ein Reservekohlenhydrat, das von vielen Pflanzen in Form von üblicherweise 1 bis 200 μm großen Stärkekörnern (Granula) in verschiedenen Pflanzenteilen gespeichert wird, z.B. in Knollen oder Wurzeln, Getreidesamen, Früchten sowie im Mark. Eine erfindungsgemäß verwendbare nichtionische, mittels Propylenoxid modifizierte Stärke kann sich aus Stärke von Kartoffeln, Mais, Reis, Erbsen, Eicheln, Kastanien, Gerste, Weizen, Bananen, Sago, Hirse, Sorghum, Hafer, Gerste, Roggen, Bohnen, Batate, Maranta oder Maniok ableiten. Besonders ausgeprägte erfindungsgemäße Effekte werden durch nichtionische, mittels Propylenoxid modifizierte Tapioka Stärke bzw. nichtionische, mittels Propylenoxid modifizierte Kartoffelstärke bzw. durch Gemische beider vorgenannter Stärken, erzielt. Ganz besonders bevorzugt enthält das erfindungsgemäße Mittel mindestens eine nichtionische, mittels Propylenoxid modifizierte Kartoffelstärke.

Stärke gehört zu der Familie der Homoglycane und ist ein Polykondensationsprodukt von D-Glucose. Dabei besteht Stärke aus drei strukturell verschiedenen Polymeren der d-Glucopyranose, nämlich der Amylose, dem Amylopektin und einer sogenannten Zwischenfraktion. Höhere Pflanzen enthalten 0 bis 45 Gew.-% Amylose bezogen auf die Trockensubstanz.

Die Zwischenfraktion, die auch als anormales Amylopektin bezeichnet wird, steht strukturell zwischen der Amylose und dem Amylopektin. Die im Rahmen dieser Anmeldung definierten Mengenangaben für Amylopektin umfassen die Zwischenfraktion.

Es ist erfindungsgemäß bevorzugt, wenn die nichtionische, mittels Propylenoxid modifizierte Stärke einen Amylosegehalt von kleiner 25 Gew.-%, insbesondere von kleiner 20 Gew.-%, - jeweils bezogen auf das Gewischt der modifizierten Stärke - besitzt. Es zeigte sich, dass sich zur Erreichung des erfindungsgemäßen Effektes besonders eine Stärke eignet, die 17 bis 22 Gew.-% Amylose und 78 bis 83 Gew.-% Amylopektin enthält.

Amylose besteht aus überwiegend linear α-1,4-glycosidisch verknüpfter d-Glucose, $M_r$ 50000-150000. Die resultierenden Ketten bilden in der Stärke Doppelhelices.

Amylopektin enthält neben den für Amylose beschriebenen α-1,4-Verknüpfungen auch in einer Menge von 4 bis 6% α-1,6-Bindungen als Verzweigungsstellen. Der durchschnittliche Abstand zwischen den Verzweigungsstellen beträgt etwa 12 bis 17 Glucose-Einheiten. Die Molmasse von $10^7$ bis $7 \cdot 10^8$ entspricht ca. $10^5$ Glucose-Einheiten, womit Amylopektin zu den größten Biopolymeren gehört. Besagte Verzweigungen sind derart über das Molekül verteilt, daß sich eine Büschelstruktur mit relativ kurzen Seitenketten entwickelt. Zwei dieser Seitenketten bilden jeweils eine Doppelhelix. Durch die vielen Verzweigungsstellen ist Amylopektin in Wasser relativ gut löslich.

Unter einer nichtionischen, mittels Propylenoxid modifizierten Stärke wird erfindungsgemäß ein Reaktionsprodukt aus einer Stärke und Propylenoxid verstanden. Ein solches Reaktionsprodukt umfasst mindestens eine Struktureinheit der Formel (PS),

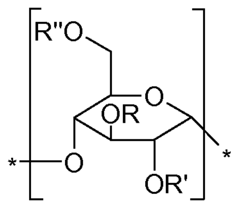

(PS),

worin mindestens ein Rest R, R' oder R" für eine Gruppe der Formel

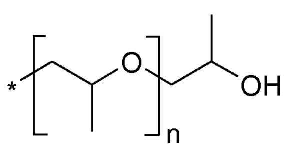

mit n ≥0 steht und maximal 2 der Reste aus R, R', R" für ein Wasserstoffatom steht. Eine mit dem Symbol * gekennzeichnete Bindung in Formeln dieser Anmeldung entspricht einer freien Valenz der entsprechenden Struktureinheit. Die nichtionischen, mittels Propylenoxid modifizierten Stärken werden beispielsweise durch Umsetzung einer nativen Stärke mit Propylenoxid bereitgestellt. Vor der Modifikation mit Propylenoxid kann die Stärke diversen physikalischen oder chemischen Prozessen ausgesetzt worden sein, wie z.B. einer Wärmebehandlung, Scherung, einer thermischen, säurehydrolytischen, oxidativen oder enzymatischen Spaltung, etc.

Es ist erfindungsgemäß bevorzugt, wenn die nichtionische, mittels Propylenoxid modifizierte Stärke in dem erfindungsgemäßen Mittel nicht in Form einzelner Stärkekörnchen (Granula) vorliegt. Zu diesem Zweck werden die Stärkekörner aufgeschlossen z.B. durch Hitze oder Scherung und die entsprechenden Polysaccharidmoleküle aus dem Verbund freigesetzt. Die freigesetzten Polysaccharidmoleküle werden nach oder vor der Freisetzung mittels Propylenoxid modifiziert.

Im Rahmen einer bevorzugten Ausführungsform ist die nichtionische, mittels Propylenoxid modifizierte Stärke verkleistert. Wird eine wässrige Suspension von Stärke erhitzt oder komprimiert, so beobachtet man bei einer kritischen Temperatur bzw. Druck eine tangentiale Quellung der Körper mit Verlust der Doppelbrechung, Änderung der Röntgenstruktur und abrupter Steigerung der Viskosität der Lösung. Diese Erscheinung wird Verkleisterung genannt.

Die erfindungsgemäßen nichtionischen, mittels Propylenoxid modifizierten Stärken liegen in dem erfindungsgemäßen Mittel in einer Molekulargewichtsverteilung vor. Die Molekulargewichtsverteilung wurde experimentell mittels Gelfiltrationschromatographie gegen Dextran bestimmt. Das besagte Gewichtsmittel ist ein mittleres Molekulargewicht, welches das Totalgewicht der Moleküle verschiedenen Molekulargewichts berücksichtigt und nicht lediglich nur die Anzahl der Moleküle. Zur statistischen Berechnung des Gewichtsmittels wird zunächst der "Gewichtsbruch"

$$w_i = (N_i\, M_i) / [\Sigma(N_i\, M_i)]$$

definiert. Dieser gibt den Gewichtsanteil an Makromolekülen in der Probe an, die aus i Segmenten (z. B. Monomerbausteinen) der Masse $M_i$ bestehen und in der Probe $N_i$-mal vorkommen. Für das Gewichtsmittel des Molekulargewichts $M_w = \Sigma w_i\, M_i$ gilt damit

$$M_w = [\Sigma(N_i\, M^2_i)] / [\Sigma(N_i\, M_i)].$$

Erfindungsgemässe Mittel enthalten solche nichtionischen, mittels Propylenoxid modifizierten Stärken, die ein mittleres Molekulargewicht (Gewichtsmittel) von 700 bis 1000 kDa, aufweisen.

Zur Einstellung des Molekulargewichts wird die Stärke vor oder nach der Modifizierung mittels Propylenoxid einer mechanischen und/oder einer chemischen Behandlung unterzogen. Zur Molekulargewichtserhöhung kann die besagte Stärke vernetzt werden. Eine Vernetzung der nichtionischen, mittels Propylenoxid modifizierten Stärke liegt vor, wenn die linearen bzw. verzweigten Polysaccharid-Makromoleküle der Stärke kovalent durch ein Vernetzungsmittel unter Bildung eines dreidimensionalen, unlöslichen und nur noch quellbaren polymeren Netzwerkes verknüpft werden. Native Stärke gilt im Allgemeinen als unvernetzt und bedarf - falls eine Vernetzung gewünscht wäre - einer künstlichen Vernetzung mittels Synthesechemie. Eine solche künstliche Vernetzung lässt sich mit Vernetzungsmitteln durchführen. (Nichtionische, mittels Propylenoxid modifizierte) Stärken, die eine solche Vernetzung nicht aufweisen, sind unvernetzt. Eine Vernetzung erfolgt beispielsweise durch das Vernetzungmittel Epichlorhydrin. Hierzu wird eine 42 Gew.-%-ige Mischung von nichtionischer, mittels Propylenoxid modifizierter Stärke in Wasser hergestellt, in diese die gewünschte Menge Epichlorhydrin bei Raumtemperatur eingerührt und nach einer Rührzeit von 1 bis 5 Stunden unter Kontrolle der Viskosität nach erreichen der Zielviskosität die vernetzte Stärke nach gängigen Verfahren isoliert.

Es ist jedoch erfindungsgemäß besonders bevorzugt, wenn das erfindungsgemäße Mittel als nichtionische, mittels Propylenoxid modifizierte Stärke mindestens eine unvernetzte, nichtionische, mittels Propylenoxid modifizierte Stärke enthält.

Zur Erzielung eines niedrigeren Molekulargewichts von 100 bis 400 kDa, werden besagte Stärken bevorzugt einer mechanischen Spaltung, einer enzymatischen Spaltung (insbesondere mit alpha-Amylase, beta-Amylase, Glucoamylase oder entzweigende Enzyme), einer säurehydrolytischen Spaltung (insbesondere mit Salzsäure, Schwefelsäure oder Phosphorsäure), einer thermischen Spaltung oder einer Umsetzung mit Oxidationsmitteln (wie Periodat, Hypochlorit, Chromsäure, Permanganat, Stickstoffdioxid, Wasserstoffperoxid oder organischer Percarbonsäure, bevorzugt mit Wasserstoffperoxid), ausgesetzt. Zur mechanischen Spaltung der Stärke eignen sich Kneter, extruder, Stator/Rotor-Maschinen und/oder Rührwerke.

Die oxidative Spaltung mittels Wasserstoffperoxid ist erfindungsgemäß bevorzugt. Zu diesem Zweck wird beispielsweise die nichtionische, mittels Propylenoxid modifizierte Stärke in Wasser gegeben, auf 50 bis 70 °C erhitzt, Wasserstoffperoxid zugefügt und bei 70 bis 85°C für 2 bis 5 Stunden gerührt.

Der Gehalt an Propylenoxid der Stärke wirkt sich auf die Feinabstimmung des Frisurenhalts mit der Frisurenflexibilität und der Stabilität der kosmetischen Mittel aus. Es zeigte sich, dass die Parameter weiter optimiert werden, wenn die nichtionische, mittels Propylenoxid modifizierte Stärke - bezogen auf das Gewicht der modifizierten Stärke - einen Propylenoxidgehalt von 1 bis 20 Gew.-%, besonders bevorzugt einen Propylenoxidgehalt von 4 bis 12 Gew.-%,

ganz besonders bevorzugt einen Propylenoxidgehalt von 9,5 bis 10,5 Gew.-% oder von 4,0 bis 6,0 Gew.-%, aufweist. Der Propylenoxidgehalt lässt sich beispielsweise nach Durchführung einer Hodges-Spaltung mit der Methode gemäß DIN EN 13268 bestimmen.

Ferner hat es sich erwiesen, dass sich solche kosmetischen Mittel im Sinne der Erfindung hervorragend eignen, in denen die nichtionische, mittels Propylenoxid modifizierte Stärke in einer 43 Gew.-%-igen wässrigen Lösung eine bevorzugte Viskosität im Bereich von 150 bis 1500000 mPa·s (Brookfield Viskosimeter, Spindel #7 bei 20°C und 20 U/min) aufweist. Besonders geeignete mittels Propylenoxid modifizierte Stärken weisen Viskositäten von 10000 bis 200000 mPa·s, besonders bevorzugt von 25000 bis 180000 mPa·s, (jeweils gemessen unter den zuvor genannten Bedingungen) auf.

Es ist erfindungsgemäß bevorzugt, wenn das kosmetisches Mittel die nichtionische, mittels Propylenoxid modifizierte Stärke in einer Menge von 0,1 Gew.-% bis 10 Gew.-%, besonders bevorzugt von 0,2 Gew.-% bis 5,0 Gew.-%, ganz besonders bevorzugt von 1,0 bis 3,0 Gew.-%, jeweils bezogen auf das Gewicht des Mittels, enthält.

Die erfindungsgemäßen Mittel enthalten ihre Wirkstoffe in einem kosmetischen Träger, bevorzugt in einem wasserhaltigen kosmetischen Träger, alkoholischen kosmetischen Träger oder einem wässrig-alkoholischen kosmetischen Träger. Zum Zwecke der temporären Haarumformung sind solche Träger beispielsweise Lotionen, Wasser-in-Öl-Emulsionen, Öl-in-Wasser-Emulsionen, Cremes, Gele, Schäume, Pomaden, Wachse oder andere Zubereitungen, die für die Anwendung auf dem Haar geeignet sind. Es ist erfindungsgemäß bevorzugt, wenn es sich um einen wasserhaltigen kosmetischen Träger oder einen wässrig-alkoholischen kosmetischen Träger handelt. Es ist erfindungsgemäß bevorzugt, wenn der kosmetische Träger des erfindungsgemäßen Mittels Wasser enthält, so dass das erfindungsgemäße Mittel mindestens 50 Gew.-% Wasser - bezogen auf das Gewicht des gesamten Mittels - enthält.

Unter wässrig-alkoholischen Trägern sind im Sinne der vorliegenden Erfindung wässrige Zusammensetzungen enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol, 1,2-Propylenglykol oder 1,3-Propylenglykol enthalten. Bevorzugt sind dabei alle wasserlöslichen organischen Lösemittel.

Es ist erfindungsgemäß bevorzugt, wenn das erfindungsgemäße Mittel zusätzlich mindestens ein filmbildendes und/oder festigendes Polymer enthält. Die zusätzlichen filmbildenden und/oder festigenden Polymere dieser Ausführungsform des erfindungsgemäßen Mittels sind von den Chitosan(derivat)en und den nichtionischen, mittels Propylenoxid modifizierten Stärken verschieden. Die filmbildenden und/oder festigenden Polymere können dabei sowohl nichtionisch, anionisch, amphoter oder kationisch geladen, bevorzugt nichtionisch oder kationisch geladen, sein.

Unter Polymeren werden erfindungsgemäß Verbindungen verstanden, die aus einer Vielzahl von Molekülen aufgebaut sind, in denen eine Art oder mehrere Arten von Atomen oder Atomgruppierungen (sogenannte konstitutive Einheiten, Grundbausteine oder Wiederholungseinheiten) wiederholt aneinander gereiht sind und die ein Molekulargewicht von wenigstens 10000 g/mol besitzen. Die Polymere werden durch Polyreaktion erhalten, wobei letztere künstlich (d.h. synthetisch) oder natürlich erfolgen kann.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser, Alkohol oder Wasser/Alkohol-Gemischen besitzen. So lassen sich entsprechende Lösungen herstellen, die sich auf einfache Art und Weise anwenden bzw. weiterverarbeiten lassen.

Unter filmbildenden Polymeren werden weiterhin solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Die zusätzlichen filmbildenden und/oder festigenden Polymere sind bevorzugt in einer Menge von 0,1 Gew.-% bis 10 Gew.-%, insbesondere von 1,0 Gew.-% bis 8,0 Gew.-%, ganz besonders bevorzugt von 2,0 bis 6,0 Gew.-%, jeweils bezogen auf das Gewicht des Mittels, enthalten.

Im Rahmen einer besonders bevorzugten Ausführungsform enthalten die erfindungsgemäßen Mittel als zusätzliches filmbildendes und/oder festigendes Polymer mindestens ein filmbildendes nichtionisches und/oder festigendes nichtionisches Polymer.

Die zusätzlichen filmbildenden nichtionischen und/oder festigenden nichtionischen Polymere sind in dem erfindungsgemäßen Mittel bevorzugt in einer Menge von 0,1 Gew.-% bis 10,0 Gew.-%, besonders bevorzugt von 1,0 Gew.-% bis 8,0 Gew.-%, ganz besonders bevorzugt von 2,0 Gew.-% bis 6,0 Gew.-%, jeweils bezogen auf das Gewicht der erfindungsgemäßen Mittel, enthalten.

Die filmbildenden nichtionischen und/oder festigenden nichtionischen Polymere werden wiederum bevorzugt ausgewählt aus mindestens einem Polymer der Gruppe, die gebildet wird, aus

- Homopolymeren und nichtionischen Copolymeren des N-Vinylpyrrolidons,
- nichtionischen Copolymeren des Isobutens,
- nichtionischen Copolymeren des Maleinsäureanhydrids.

Geeignete Polyvinylpyrrolidone sind beispielsweise Handelsprodukte wie Luviskol® K 90 oder Luviskol® K 85 der Firma BASF SE.
Geeignetes Polyvinylacetat wird beispielsweise unter dem Handelsnamen Vinac® als Emulsion von der Firma Air Products vertrieben.
Mittel, die als filmbildendes nichtionisches und/oder festigendes nichtionisches Polymer mindestens ein Polymer ausgewählt aus der Gruppe, die gebildet wird aus

- Copolymer aus Maleinsäureanhydrid und Methylvinylether,
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
  oder Gemische aus diesen Polymeren enthalten, sind erfindungsgemäß ganz besonders bevorzugt. Dabei sind wiederum solche erfindungsgemäßen Mittel bevorzugt, die als filmbildendes nichtionisches und/oder festigendes nichtionisches Polymer mindestens ein Polymer ausgewählt aus der Gruppe, die gebildet wird aus

- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,

oder Gemische aus diesen Polymeren enthalten.
Wenn Copolymere aus N-Vinylpyrrolidon und Vinylestern von Carbonsäuren mit 2 bis 18 Kohlenstoffatomen, insbesondere aus N-Vinylpyrrolidon und Vinylacetat eingesetzt werden, ist es wiederum bevorzugt, wenn das Molverhältnis der aus dem Monomer N-Vinylpyrrolidon enthaltenen Struktureinheiten zu den aus dem Monomer Vinylestern von Carbonsäuren mit 2 bis 18 Kohlenstoffatomen (insbesondere Vinylacetat) enthaltenen Struktureinheiten des Polymers im Bereich von 20 zu 80 bis 80 zu 20, insbesondere von 30 zu 70 bis 60 zu 40, liegt.
Geeignete Copolymerisate aus Vinylpyrrolidon und Vinylacetat sind beispielsweise unter dem Warenzeichen Luviskol® VA 37, Luviskol® VA 55, Luviskol® VA 64 und Luviskol® VA 73 von der Firme BASF SE erhältlich.
Die erfindungsgemäßen Mittel enthalten bevorzugt als zusätzliches filmbildendes und/oder festigendes Polymer mindestens ein kationisches, filmbildendes und/oder kationisch, festigendes Polymer. Hierbei ist es wiederum erfindungsgemäß bevorzugt, wenn als zusätzliches filmbildendes und/oder festigendes Polymer mindestens eine Kombination aus

- mindestens einem nichtionischen, filmbildenden und/oder nichtionisch, festigenden Polymer und
- mindestens einem kationischen, filmbildenden und/oder kationischen, festigenden Polymer in dem erfindungsgemäßen Mittel enthalten ist.

Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.
Ein erfindungsgemäß bevorzugt geeignetes kationisches filmbildendes und/oder kationisches festigendes Polymer ist mindestens ein kationisches filmbildendes und/oder kationisches festigendes Polymer, das mindestens ein Strukturelement der Formel (M9) und zusätzlich mindestens ein Strukturelement der Formel (M10) enthält

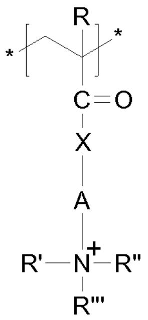 (M9) 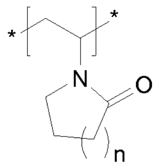 (M10)

worin

| | |
|---|---|
| R | für ein Wasserstoffatom oder eine Methylgruppe steht, |
| R', R" und R" | unabhängig voneinander stehen für eine ($C_1$ bis $C_{30}$)-Alkylgruppe, |
| X | steht für ein Sauerstoffatom oder eine Gruppe NH, |
| A | steht für eine Ethan-1,2,-diylgruppe oder eine Propan-1,3-diylgruppe, |
| n | 1 oder 3 bedeutet. |

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Solche Verbindungen sind beispielsweise als

- Copolymere aus mit Diethylsulfat quaterniertem Dimethylaminoethylmethacrylat, mit Vinylpyrrolidon mit der INCI-Bezeichnung Polyquaternium-11 unter den Bezeichnungen Gafquat® 440, Gafquat®734, Gafquat®755 (jeweils Firma ISP) sowie Luviquat PQ 11 PN (Firma BASF SE),
- Copolymere aus N-Vinylpyrrolidon, N-(3-Dimethylaminopropyl)methacrylamid und 3-(Methacryloylamino)propyl-lauryl-dimethylammoniumchlorid (INCI-Bezeichnung: Polyquaternium-55), welches beispielsweise unter dem Handelsnamen Styleze W 10 oder W 20 (10 bzw. 20 Gew.-% Aktivsubstanz in Ethanol-Wasser-Gemisch) von der Firma ISP vertrieben wird.
- Copolymere aus N-Vinylpyrrolidon, N-Vinylcaprolactam, N-(3-Dimethylaminopropyl)methacrylamid und 3-(Methacryloylamino)propyl-lauryldimethylammoniumchlorid (INCI-Bezeichnung: Polyquaternium-69), welches beispielsweise unter dem Handelsnamen AquaStyle® 300 (28-32 Gew.-% Aktivsubstanz in Ethanol-Wasser-Gemisch) von der Firma ISP vertrieben wird.

Weiterhin werden die kationischen filmbildenden und/oder kationischen festigenden Polymere erfindungsgemäß besonders bevorzugt aus kationischen, quaternisierten Cellulose-Derivaten ausgewählt.

Ferner eignen sich als filmbildendes und/oder festigendes Polymere bevorzugt kationische, quaternisierte Cellulosederivate.

Es erweisen sich solche kationischen, quaternisierten Cellulosen als im Sinne der Erfindung besonders vorteilhaft, die in einer Seitenkette mehr als eine permanente kationische Ladung tragen. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat® H 100, Celquat® L 200 von der Firma National Starch vertrieben werden.

Als im Sinne der Erfindung besonders bevorzugt verwendbare kationische Polymere dienen weiterhin solche kationischen filmbildenden und/oder kationischen festigenden Copolymere, die mindestens ein Strukturelement der Formel (M11)

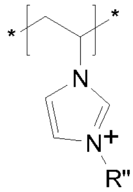 (M11)

worin R" für eine ($C_1$ bis $C_4$)-Alkylgruppe, insbesondere eine Methylgruppe, steht, und zusätzlich mindestens ein weiteres kationisches und/oder nichtionisches Strukturelement aufweisen.

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Es ist wiederum erfindungsgemäß bevorzugt, wenn als zusätzliches kationisches filmbildendes und/oder kationisches festigendes Polymer, mindestens ein Copolymer (c1) enthalten ist, das neben mindestens einem Strukturelement der Formel (M11) zusätzlich ein Strukturelement der Formel (M6) umfasst

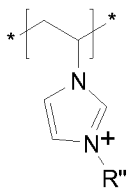
(M11)
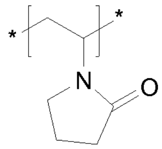
(M6).

worin R" für eine ($C_1$ bis $C_4$)-Alkylgruppe, insbesondere eine Methylgruppe, steht.

Zur Kompensation der positiven Polymerladung der Copolymere (c1) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Ganz besonders bevorzugte kationische filmbildende und/oder kationische festigende Polymere als Copolymere (c1) enthalten 10 bis 30 Mol-%, vorzugsweise 15 bis 25 Mol.-% und insbesondere 20 Mol.-% Struktureinheiten gemäß Formel (M11) und 70 bis 90 Mol.-%, vorzugsweise 75 bis 85 Mol.-% und insbesondere 80 Mol.-% Struktureinheiten gemäß Formel (M6).

Hierbei ist besonders bevorzugt, wenn die Copolymere (c1) neben Polymereinheiten, die aus dem Einbau der genannten Struktureinheiten gemäß Formel (M11) und (M6) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c1) ausschließlich aus Struktureinheiten der Formel (M11) mit R" = Methyl und (M6) aufgebaut.

Wird in zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat® Style, Luviquat® FC 370, Luviquat®FC 550, Luviquat®FC 905 und Luviquat® HM 552

Wird in zur Kompensation der positiven Ladung des Polymers der Formel (Poly1) ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der BASF unter den Handelsnamen Luviquat® UltraCare erhältlich.

Zusätzlich zu dem bzw. den Copolymer(en) (c1) oder an dessen bzw. deren Stelle können die erfindungsgemäßen Mittel auch Copolymere (c2) enthalten, die ausgehend vom Copolymer (c1) als zusätzliche Struktureinheiten Struktureinheiten der Formel (M7) aufweisen

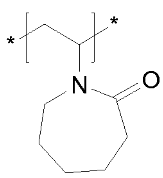
(M7).

Weitere besonders bevorzugte erfindungsgemäße Mittel sind somit dadurch gekennzeichnet, daß sie als kationisches filmbildendes und/oder kationisches festigendes Polymer mindestens ein Copolymer (c2) enthalten, das mindestens eine Struktureinheit gemäß Formel (M11-a) und mindestens eine Struktureinheit gemäß Formel (M6) und mindestens eine Struktureinheit gemäß Formel (M7) enthält

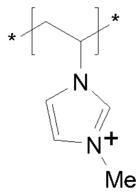 (M11-a) 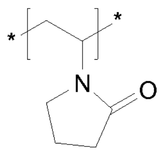 (M6) 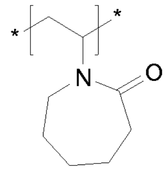 (M7)

Auch hierbei ist besonders bevorzugt, wenn die Copolymere (c2) neben Polymereinheiten, die aus dem Einbau der genannten Struktureinheiten gemäß Formel (M11-a), (M6) und (M7) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c2) ausschließlich aus Struktureinheiten der Formeln (M11-a), (M6) und (M7) aufgebaut.

Zur Kompensation der positiven Polymerladung der Komponente (c2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly2) ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat® Hold erhältlich.

Ganz besonders bevorzugte Copolymere (c2) enthalten 1 bis 20 Mol-%, vorzugsweise 5 bis 15 Mol.-% und insbesondere 10 Mol.-% Struktureinheiten gemäß Formel (M11-a) und 30 bis 50 Mol.-%, vorzugsweise 35 bis 45 Mol.-% und insbesondere 40 Mol.-% Struktureinheiten gemäß Formel (M6) und 40 bis 60 Mol.-%, vorzugsweise 45 bis 55 Mol.-% und insbesondere 60 Mol.-% Struktureinheiten gemäß Formel (M7).

Zusätzlich zu dem bzw. den Copolymer(en) (c1) und/oder (c2) oder an dessen bzw. deren Stelle können die erfindungsgemäßen Mittel als filmbildendes kationische und/oder festigendes kationisches Polymer auch Copolymere (c3) enthalten, die als Struktureinheiten Struktureinheiten der Formeln (M11-a) und (M6) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Weitere besonders bevorzugte erfindungsgemäße Mittel sind dadurch gekennzeichnet, daß sie als zusätzliches kationisches filmbildendes und/oder kationisches festigendes Polymer mindestens ein Copolymer (c3) enthalten, das mindestens eine Struktureinheit gemäß Formel (M11-a) und mindestens eine Struktureinheit gemäß Formel (M6) und mindestens eine Struktureinheit gemäß Formel (M10) und mindestens eine Struktureinheit gemäß Formel (M12) enthält

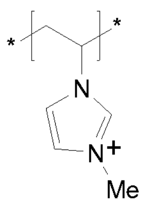 (M11-a) 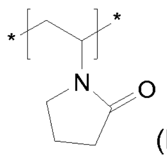 (M6) 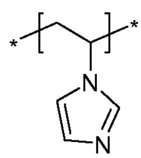 (M8) 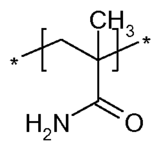 (M12).

Auch hierbei ist besonders bevorzugt, wenn die Copolymere (c3) neben Polymereinheiten, die aus dem Einbau der genannten Struktureinheiten gemäß Formel (M11-a), (M6), (M8) und (M12) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c3) ausschließlich aus Struktureinheiten der Formel (M11-a), (M6), (M8) und (M12) aufgebaut.

Zur Kompensation der positiven Polymerladung der Komponente (c3) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat® Supreme erhältlich.

Ganz besonders bevorzugte Copolymere (c3) enthalten 1 bis 12 Mol-%, vorzugsweise 3 bis 9 Mol.-% und insbesondere 6 Mol.-% Struktureinheiten gemäß Formel (M11-a) und 45 bis 65 Mol.-%, vorzugsweise 50 bis 60 Mol.-% und insbesondere 55 Mol.-% Struktureinheiten gemäß Formel (M6) und 1 bis 20 Mol.-%, vorzugsweise 5 bis 15 Mol.-% und insbesondere 10 Mol.-% Struktureinheiten gemäß Formel (M8) und 20 bis 40 Mol.-%, vorzugsweise 25 bis 35 Mol.-% und insbesondere 29 Mol.-% Struktureinheiten gemäß Formel (M12).

Unter den zusätzlichen filmbildenden kationischen und/oder festigenden Polymer ausgewählt aus den kationischen Polymeren mit mindestens einem Strukturelement der obigen Formel (M11-a), gelten als bevorzugt:

- Vinylpyrrolidon/1-Vinyl-3-methyl-1H-imidazoliumchlorid-Copolymere (wie beispielsweise das mit der INCI-Bezeichnung Polyquaternium-16 unter den Handelsbezeichnungen Luviquat® Style, Luviquat® FC 370, Luviquat® FC 550, Luviquat® FC 905 und Luviquat® HM 552 (BASF SE)),
- Vinylpyrrolidon/1-Vinyl-3-methyl-1H-imidazoliummethylsulfat-Copolymere (wie beispielsweise das mit der INCI-Bezeichnung Polyquaternium-44 unter den Handelsbezeichnungen Luviquat® Care (BASF SE)),
- Vinylpyrrolidon/Vinylcaprolactam/1-Vinyl-3-methyl-1 H-imidazolium-Terpolymer (wie beispielsweise das mit der INCI-Bezeichnung Polyquaternium-46 unter den Handelsbezeichnungen Luviquat® Care oder Luviquat® Hold (BASF SE)),
- Vinylpyrrolidon/Methacrylamid/Vinylimidazol/1-Vinyl-3-methyl-1H-imidazoliummethylsulfat-Copolymer (wie beispielsweise das mit der INCI-Bezeichnung Polyquaternium-68 unter der Handelsbezeichnung Luviquat® Supreme (BASF SE)),

sowie Gemische aus diesen Polymeren.

Weitere in den erfindungsgemäßen Mitteln bevorzugt einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Als im Sinne der Erfindung bevorzugt geeignete temporär kationische Polymere gelten gleichfalls solche, die mindestens eine Struktureinheit der Formeln (M1-1) bis (M1-8) aufweisen

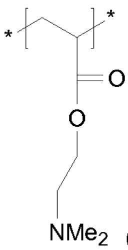 (M1-1) 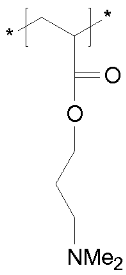 (M1-2) 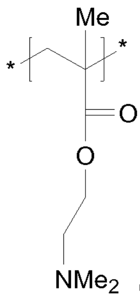 (M1-3) 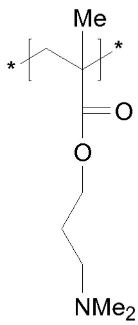 (M1-4)

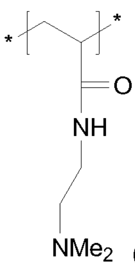 (M1-5) 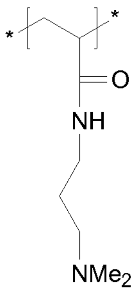 (M1-6) 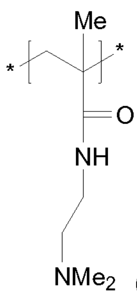 (M1-7) 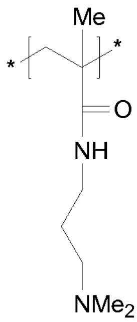 (M1-8).

Dabei sind wiederum solche Copolymere bevorzugt, die mindestens eine Struktureinheit der Formeln (M1-1) bis (M1-8) und zusätzlich mindestens eine Struktureinheit der Formel (M10) enthalten,

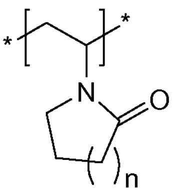 (M10)

worin

n 1 oder 3 bedeutet.

Dabei gilt wiederum die Gruppe der Polymere

- Vinylcaprolactam/Vinylpyrrolidon/Dimethylaminoethylmethacrylat-Copolymer (beispielsweise INCI-Bezeichnung: Vinyl Caprolactam/PVP/Di-methylaminoethyl Methacrylate Copolymer unter dem Handelsnamen Gaffix® VC 713 (ISP)),
- Vinylpyrrolidon/Vinylcaprolactam/Dimethylaminopropylmethacrylamid-Copolymer (beispielsweise INCI-Bezeichnung: VP/Vinyl Caprolactam/DMAPA Acrylates Copolymer unter dem Handelsnamen Aquaflex® SF-40 (ISP)),
- Vinylcaprolactam/Vinylpyrrolidon/Dimethylaminoethylmethacrylat-Copolymer (beispielsweise als.35-39% Festkörper in Ethanol in form des Handelsprodukts Advantage LC E mit der INCI-Bezeichnung: Vinyl Caprolactam/VP/Dimethylaminoethyl Methacrylate Copolymer, Alcohol, Lauryl Pyrrolidone (ISP)),
- Vinylpyrrolidon/Dimethylaminopropylmethacrylamid-Copolymer (beispielsweise INCI-Bezeichnung: VP/DMAPA Acrylates Copolymer unter dem Handelsnamen Styleze CC-10 (ISP)),

als bevorzugte Liste zur Auswahl mindestens eines oder mehrerer Polymere daraus.

Erfindungsgemäß ganz besonders bevorzugte kosmetische Mittel gehorchen mindestens einer der folgenden Ausführungsformen

Kosmetisches Mittel zur temporären Umformung keratinischer Fasern, insbesondere menschlicher Haare, enthaltend in einem kosmetischen Träger

- mindestens ein Neutralisationsprodukt von Chitosan mit mindestens einer organischen Carbonsäure und
- mindestens eine nichtionische, mittels Propylenoxid modifizierte Stärke mit einem Molekulargewicht (Gewichtsmittel) von 700 bis 1000 kDa.

Kosmetisches Mittel zur temporären Umformung keratinischer Fasern, insbesondere menschlicher Haare, enthaltend in einem kosmetischen Träger

- mindestens ein Neutralisationsprodukt von Chitosan mit mindestens einer organischen Carbonsäure,
- mindestens eine nichtionische, mittels Propylenoxid modifizierte Stärke mit einem Molekulargewicht (Gewichtsmittel) von 700 bis 1000 kDa und
- mindestens ein nichtionisches, filmbildendes und/oder nichtionisches festigendes Polymer. Kosmetisches Mittel zur temporären Umformung keratinischer Fasern, insbesondere menschlicher Haare, enthaltend in einem kosmetischen Träger
- mindestens ein Neutralisationsprodukt von Chitosan mit mindestens einer organischen Carbonsäure,
- mindestens eine nichtionische, mittels Propylenoxid modifizierte Stärke mit einem Molekulargewicht (Gewichtsmittel) von 700 bis 1000 kDa und
- mindestens ein nichtionisches, filmbildendes und/oder nichtionisches festigendes Polymer, ausgewählt aus der Gruppe, die gebildet wird aus
- Copolymer aus Maleinsäureanhydrid und Methylvinylether,
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,

oder Gemische aus diesen Polymeren.

Kosmetisches Mittel zur temporären Umformung keratinischer Fasern, insbesondere menschlicher Haare, enthaltend in einem kosmetischen Träger

- mindestens ein Neutralisationsprodukt von Chitosan mit mindestens einer organischen Carbonsäure,
- mindestens eine nichtionische, mittels Propylenoxid modifizierte Stärke mit einem Molekulargewicht (Gewichtsmittel) von 700 bis 1000 kDa und einer Viskosität im Bereich von 150
  bis 1500000 mPa·s, bevorzugt von 10000 bis 200000 mPa·s, besonders bevorzugt von 25000 bis 180000 mPa·s, (jeweils in einer 43 Gew.-%-igen wässrigen Lösung, Brookfield Viskosimeter, Spindel #7 bei 20°C und 20 U/min), und
- mindestens ein nichtionisches, filmbildendes und/oder nichtionisches festigendes Polymer. Kosmetisches Mittel zur temporären Umformung keratinischer Fasern, insbesondere menschlicher Haare, enthaltend in einem kosmetischen Träger
- mindestens ein Neutralisationsprodukt von Chitosan mit mindestens einer organischen Carbonsäure,
- mindestens eine nichtionische, mittels Propylenoxid modifizierte Stärke mit einem Molekulargewicht (Gewichtsmittel)

von 700 bis 1000 kDa und einer Viskosität im Bereich von 150 bis 1500000 mPa·s, bevorzugt von 10000 bis 200000 mPa·s, besonders bevorzugt von 25000 bis 180000 mPa·s, (jeweils in einer 43 Gew.-%-igen wässrigen Lösung, Brookfield Viskosimeter, Spindel #7 bei 20°C und 20 U/min), und

- mindestens ein nichtionisches, filmbildendes und/oder nichtionisches festigendes Polymer, ausgewählt aus der Gruppe, die gebildet wird aus
  - Copolymer aus Maleinsäureanhydrid und Methylvinylether,
  - Polyvinylpyrrolidon,
  - Copolymeren aus N-Vinylpyrrolidon und Vinylestern von Carbonsäuren mit 2 bis 18 Kohlenstoffatomen, insbesondere aus N-Vinylpyrrolidon und Vinylacetat,

  oder Gemische aus diesen Polymeren.

Kosmetisches Mittel zur temporären Umformung keratinischer Fasern, insbesondere menschlicher Haare, enthaltend in einem kosmetischen Träger

- mindestens ein Neutralisationsprodukt von Chitosan mit mindestens einer organischen Carbonsäure und
- mindestens eine nichtionische, mittels Propylenoxid modifizierte Stärke mit einem Molekulargewicht (Gewichtsmittel) von 700-1000 kDa mit einer Viskosität im Bereich von 150 bis 1500000 mPa·s, bevorzugt von 10000 bis 200000 mPa·s, besonders bevorzugt von 25000 bis 180000 mPa·s, (jeweils in einer 43 Gew.-%-igen wässrigen Lösung, Brookfield Viskosimeter, Spindel #7 bei 20°C und 20 U/min) und einem Propylenoxidgehalt von 1 bis 20 Gew.-%, bevorzugt einen Propylenoxidgehalt von 4 bis 12 Gew.-%, besonders bevorzugt einen Propylenoxidgehalt von 9,5 bis 10,5 Gew.-% oder von 4,0 bis 6,0 Gew.-%, - jeweils bezogen auf das Gewicht der modifizierten Stärke.

Kosmetisches Mittel zur temporären Umformung keratinischer Fasern, insbesondere menschlicher Haare, enthaltend in einem kosmetischen Träger

- mindestens ein Neutralisationsprodukt von Chitosan mit mindestens einer organischen Carbonsäure,
- mindestens eine nichtionische, mittels Propylenoxid modifizierte Stärke mit einem Molekulargewicht (Gewichtsmittel) von 700-1000 kDa mit einer Viskosität im Bereich von 150 bis 1500000 mPa·s, bevorzugt von 10000 bis 200000 mPa·s, besonders bevorzugt von 25000 bis 180000 mPa·s, (jeweils in einer 43 Gew.-%-igen wässrigen Lösung, Brookfield Viskosimeter, Spindel #7 bei 20°C und 20 U/min) und einem Propylenoxidgehalt von 1 bis 20 Gew.-%, bevorzugt einen Propylenoxidgehalt von 4 bis 12 Gew.-%, besonders bevorzugt einen Propylenoxidgehalt von 9,5 bis 10,5 Gew.-% oder von 4,0 bis 6,0 Gew.-%, - jeweils bezogen auf das Gewicht der modifizierten Stärke -, und
- mindestens ein nichtionisches, filmbildendes und/oder nichtionisches festigendes Polymer.

Kosmetisches Mittel zur temporären Umformung keratinischer Fasern, insbesondere menschlicher Haare, enthaltend in einem kosmetischen Träger

- mindestens ein Neutralisationsprodukt von Chitosan mit mindestens einer organischen Carbonsäure,
- mindestens eine nichtionische, mittels Propylenoxid modifizierte Stärke mit einem Molekulargewicht (Gewichtsmittel) von 700-1000 kDa mit einer Viskosität im Bereich von 150 bis 1500000 mPa·s, bevorzugt von 10000 bis 200000 mPa·s, besonders bevorzugt von 25000 bis 180000 mPa·s, (jeweils in einer 43 Gew.-%-igen wässrigen Lösung, Brookfield Viskosimeter, Spindel #7 bei 20°C und 20 U/min) und einem Propylenoxidgehalt von 1 bis 20 Gew.-%, bevorzugt einen Propylenoxidgehalt von 4 bis 12 Gew.-%, besonders bevorzugt einen Propylenoxidgehalt von 9,5 bis 10,5 Gew.-% oder von 4,0 bis 6,0 Gew.-%, - jeweils bezogen auf das Gewicht der modifizierten Stärke -, und
- mindestens ein nichtionisches, filmbildendes und/oder nichtionisches festigendes Polymer, ausgewählt aus der Gruppe, die gebildet wird aus
  - Copolymer aus Maleinsäureanhydrid und Methylvinylether,
  - Polyvinylpyrrolidon,
  - Copolymeren aus N-Vinylpyrrolidon und Vinylestern von Carbonsäuren mit 2 bis 18 Kohlenstoffatomen, insbesondere aus N-Vinylpyrrolidon und Vinylacetat,

  oder Gemische aus diesen Polymeren.

Bevorzugte zusätzliche nichtionische, filmbildende und/oder nichtionische, festigende Polymere der Ausführungsformen - wenn aufgeführt - sind Polyvinylpyrrolidon, Copolymere von N-Vinylpyrrolidon und Vinylacetat oder Gemische daraus.

Bevorzugte organische Carbonsäuren des Neutralisationsproduktes von Chitosan gemäß Ausführungsformen sind ausgewählt aus Milchsäure, Ameisensäure, Pyrrolidoncarbonsäure, Nicotinsäure, Hydroxyisobuttersäure, Hydroxyisovaleriansäure oder Gemischen dieser Säuren.

Bevorzugte Einsatzmengen der Inhaltsstoffe gemäß Ausführungsformen sind die zuvor genannten (*vide supra*).

Es hat sich als bevorzugt herausgestellt, zusätzlich mindestens ein nichtionisches Tensid einzusetzen. Diese Tenside können erfindungsgemäß bereits emulgierende Wirkung haben.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 100 Mol Ethylenoxid und/oder 1 bis 5 Mol Propylenoxid an lineare oder verzweigte Fettalkohole mit 8 bis 30 Kohlenstoff-Atomen, an Fettsäuren mit 8 bis 30 Kohlenstoff-Atomen und an Alkylphenole mit 8 bis 15 C-Atomen in der Alkylgruppe,
- Anlagerungsprodukte von 2 bis 20 Einheiten Glyzerin an lineare oder verzweigte Fettalkohole mit 8 bis 30 Kohlenstoff-Atomen in der Alkylgruppe, an lineare oder verzweigte Fettsäuren mit 8 bis 30 Kohlenstoff-Atomen in der Alkylgruppe, wie beispielsweise die unter den Verkaufsbezeichnungen Dermofeel® G 10 LW (Straetmans Chemische Produkte) erhältlichen Typen,
- mit einem Methyl- oder $C_2$ - $C_6$ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 Kohlenstoff-Atomen, an Fettsäuren mit 8 bis 30 C-Atomen und an Alkylphenole mit 8 bis 15 Kohlenstoff-Atomen in der Alkylgruppe, wie beispielsweise die unter den Verkaufsbezeichnungen Dehydol® LS, Dehydol® LT (Cognis) erhältlichen Typen,
- $C_{12}$-$C_{30}$-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (E4-I)

$$R^1CO\text{-}(OCH_2CHR^2)_wOR^3 \qquad \text{(E4-I)}$$

  in der $R^1CO$ für einen linearen oder verzweigten, gesättigten und/oder ungesättigten Acylrest mit 6 bis 22 Kohlenstoffatomen, $R^2$ für Wasserstoff oder Methyl, $R^3$ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

$$R^4O\text{-}[G]_p \qquad \text{(E4-II)}$$

  in der $R^4$ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Darunter sind wiederum solche nichtionischen Tenside besonders bevorzugt zum Einsatz in dem erfindungsgemäßen Mittel geeignet, die ausgewählt werden aus

- Anlagerungsprodukten von 2 bis 20 Einheiten Glyzerin an lineare oder verzweigte Fettalkohole mit 8 bis 30 Kohlenstoff-Atomen in der Alkylgruppe,
- Anlagerungsprodukten von 2 bis 20 Einheiten Glyzerin an lineare oder verzweigte Fettsäuren mit 8 bis 30 Kohlenstoff-Atomen in der Alkylgruppe,
- Anlagerungsprodukte von 5 bis 60 Mol Ethylenoxid an Rizinusöl und gehärtetes Rizinusöl,
- Mischungen aus den vorgenannten Tensiden.

Die nichtionischen Tenside sind bevorzugt in einer Menge von 0,005 Gew.-% bis 10 Gew.-%, insbesondere von 0,01 bis 2 Gew.-%, jeweils bezogen auf das Gewicht des erfindungsgemäßen Mittels, in dem erfindungsgemäßen Mittel enthalten.

Das erfindungsgemäße Mittel weist auch ohne weitere Pflegestoffen akzeptable Haarpflegende Eigenschaften auf. Weiterhin kann das erfindungsgemäße Mittel zur Steigerung der Haarpflegewirkung zusätzlich mindestens ein kationisches Tensid enthalten. Beispiele für die in den erfindungsgemäßen Mitteln einsetzbaren kationischen Tenside sind insbesondere quartäre Ammoniumverbindungen. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, sogenannte "Esterquats", wie beispielsweise die unter den Warenzeichen Dehyquart® und Stepantex® vertriebenen Methylhydroxyalkyl-dialkoyloxyalkyl-ammoniummethosulfate, eingesetzt werden.

Die erfindungsgemäßen Mittel liegen bevorzugt als Schaum vor. Hierzu werden die erfindungsgemäßen Mittel in einer Abgabevorrichtung konfektioniert, die entweder ein zusätzlich mit einem Treibmittel befüllter Druckgasbehälter ("Aerosolbehälter") oder ein Nichtaerosolbehälter darstellt. Die Druckgasbehälter, mit deren Hilfe ein Produkt durch den inneren Gasdruck des Behälters über ein Ventil verteilt wird, bezeichnet man definitionsgemäß als "Aerosolbehälter". Als "Nichtaerosolbehälter" wird im Umkehrschluß zur Aerosoldefinition ein Behältnis unter Normaldruck definiert, mit dessen Hilfe ein Produkt mittels mechanischer Einwirkung durch ein Pump- oder Quetschsystem verteilt wird.

Insbesondere bevorzugt liegen die erfindungsgemäßen Mittel als Aerosolschaum in einem Aerosolbehälter vor. Das erfindungsgemäße Mittel enthält daher bevorzugt zusätzlich mindestens ein Treibmittel. In diesem Zusammenhang gelten insbesondere ebenso die Ausführungsformen A) bis O) als bevorzugt geeignete, in einem Aerosolbehälter mit mindestens einem Treibmittel konfektionierte, Zusammensetzungen zur Bereitstellung eines Aerosolschaumes.

Erfindungsgemäße Mittel, die in Form eines Aerosolprodukts vorliegen, lassen sich in üblicher Art und Weise herstellen. In der Regel werden alle Bestandteile des erfindungsgemäßen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

In der Ausführungsform als Aerosolschaum sind erfindungsgemäß geeignete Treibmittel beispielsweise ausgewählt aus $N_2O$ Dimethylether, $CO_2$ Luft, Alkanen mit 3 bis 5 Kohlenstoffatomen, wie Propan, n-Butan, iso-Butan, n-Pentan und iso-Pentan, und deren Mischungen. Gemäß der Ausführungsform eines Aerosolschaums werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Bevorzugt sind Dimethylether, Propan, n-Butan, iso-Butan und Mischungen daraus. Ganz besonders bevorzugt werden Mischungen von Propan und Butan als alleiniges Treibmittel verwendet im Gewichtsverhältnis Propan zu Butan von 70 zu 30 bis 15 zu 85. Diese Mischungen werden wiederum bevorzugt in den erfindungsgemäßen Mitteln in einer Menge von 3 bis 15 Gew.-% - bezogen auf das Gewicht des gesamten Mittels - eingesetzt. Unter Butan wird erfindungsgemäß n-Butan, iso-Butan und Gemische aus n-Butan und iso-Butan verstanden. Über das Mengenverhältnis von Treibmittel zu den übrigen Bestandteilen der Zubereitungen lassen sich bei gegebener Sprühvorrichtung die Größen der Schaumblasen und die jeweilige Größenverteilung einstellen.

Bei Verwendung herkömmlicher Aerosolbehälter enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis 15 Gew.-% sind besonders bevorzugt.

Zur Verschäumung von gelförmigen Mitteln in einem Zweikammer-Aerosolbehälter eignet sich bevorzugt Isopentan als ein Treibmittel, welches in die erfindungsgemäßen Mittel eingearbeitet wird und in der ersten Kammer des Zweikammer-Aerosolbehälters konfektioniert ist. In der zweiten Kammer des Zweikammer-Aerosolbehälters wird mindestens ein weiteres, von Isopentan verschiedenes Treibmittel konfektioniert, welches in dem Zweikammer-Aerosolbehälter einen höheren Druck aufbaut als das Isopentan. Die Treibmittel der zweiten Kammer werden bevorzugt ausgewählt aus $N_2O$ Dimethylether, $CO_2$, Luft, Alkanen mit 3 oder 4 Kohlenstoffatomen (wie Propan, n-Butan, iso-Butan) sowie Mischungen daraus.

Ein zweiter Gegenstand der Erfindung ist die Verwendung eines kosmetischen Mittels des ersten Erfindungsgegenstandes zur temporären Umformung und/oder Formfixierung keratinischer Fasern, insbesondere menschlicher Haare.

Ein dritter Gegenstand der Erfindung ist ein Verfahren zur temporären Umformung keratinischer Fasern, insbesondere menschlicher Haare, dadurch gekennzeichnet, dass ein kosmetisches Mittel des ersten Erfindungsgegenstandes auf die keratinischen Fasern aufgebracht wird.

Es hat sich als bevorzugt herausgestellt, wenn die keratinischen Fasern nach der Einwirkung der kosmetischen Mittel des ersten Erfindungsgegenstandes nicht gespült und auf der Faser belassen werden.

Die nachfolgenden Beispiele sollen den Gegenstand der vorliegenden Erfindung erläutern ohne ihn in irgendeiner Weise zu beschränken.

Beispiele

Folgende erfindungsgemäße Rezeptur wurde in einem Aerosolbehältnis als Aerosolschaumprodukt hergestellt:

| Rohstoff | E1 [Gew.-%] |
|---|---|
| Hydagen HCMF [1] | 0,50 |
| Milchsäure | 0,28 |
| Luviskol 60/40 W NP [2] | 10,70 |
| nichtionische, mittels Propylenoxid modifizierte Stärke [3] | 2,70 |
| Natrium Benzoat | 0,30 |
| D-Panthenol | 0,15 |
| Dow Corning 939 [4] | 0,20 |
| Dehyquart A CA [5] | 1,00 |
| Propan / Butan | 8,00 |
| Wasser | ad 100 |

[1] Chitosan (zu 80 % deacetyliert), Molekulargewicht 50000 bis 1000000 g/mol, Cognis)
[2] Copolymer aus N-Vinylpyrrolidon und Vinylacetat (
[3] Kartoffelstärke, Propylenoxidgehalt: 10 Gew.-% Propylenoxid, Viskosität: 64000 mPas, mittleres Molegulargewicht (Gewichtsmittel): 800 kDa
[4] ca. 32-36% Aktivsubstanz, INCI-Bezeichnung: Amodimethicone, Trideceth-12, Cetrimonium Chloride (Dow Corning)
[5] Trimethylhexadecylammoniumchlorid (ca. 24-26 % Aktivsubstanz; INCI-Bezeichnung: Aqua (Water), Cetrimonium Chloride) (Cognis)

Die Rezeptur E1 wurde einem Probanden zu Formfixierung der Frisur als Aerosolschaums aufgetragen. Das Haar erhielt einen natürlichen Glanz, einen starken Halt der Frisur und ein dauerhaftes Volumen.

**Patentansprüche**

1. Kosmetisches Mittel zur temporären Umformung keratinischer Fasern, insbesondere menschlicher Haare, enthaltend in einem kosmetischen Träger

   - mindestens ein Chitosan, wobei als Chitosan mindestens ein Neutralisationsprodukt von Chitosan mit mindestens einer organischen Carbonsäure enthalten ist und
   - mindestens eine nichtionische, mittels Propylenoxid modifizierte Stärke, wobei die nichtionische, mittels Propylenoxid modifizierte Stärke ein mittleres Molekulargewicht (Gewichtsmittel) von 700 bis 1000 kDa, aufweist.

2. Kosmetisches Mittel nach Anspruch 1, **dadurch gekennzeichnet, dass** die organische Carbonsäure ausgewählt wird unter Milchsäure, Ameisensäure, Pyrrolidoncarbonsäure, Nicotinsäure, Hydroxyisobuttersäure, Hydroxyisovaleriansäure oder Gemischen dieser Säuren.

3. Kosmetisches Mittel nach einem der Ansprüche 1 oder 2, **dadurch gekennzeichnet, dass** das Chitosan in einer Menge von 0,01 Gew.% bis 5 Gew.-%, bevorzugt von 0,05 Gew.% bis 2,0 Gew.-%, besonders bevorzugt von 0,1 Gew.-% bis 1 Gew.%, jeweils bezogen auf das Gewicht des erfindungsgemäßen Mittels, enthalten ist.

4. Kosmetisches Mittel nach einem der Ansprüche 1 bis 3, **dadurch gekennzeichnet, dass** die nichtionische, mittels Propylenoxid modifizierte Stärke eine nichtionische, mittels Propylenoxid modifizierte Tapioka Stärke oder eine nichtionische, mittels Propylenoxid modifizierte Kartoffelstärke oder ein Gemisch beider vorgenannter Stärken ist.

5. Kosmetisches Mittel nach einem der Ansprüche 1 bis 4, **dadurch gekennzeichnet, dass** die nichtionische, mittels Propylenoxid modifizierte Stärke einen Propylenoxidgehalt von 1 bis 20 Gew.-%, bevorzugt einen Propylenoxidgehalt von 4 bis 12 Gew.-%, besonders bevorzugt einen Propylenoxidgehalt von 9,5 bis 10,5 Gew.-% oder von 4,0 bis 6,0 Gew.-%, - jeweils bezogen auf das Gewicht der modifizierten Stärke - aufweist.

6. Kosmetisches Mittel nach einem der Ansprüche 1 bis 5, **dadurch gekennzeichnet, dass** die nichtionische, mittels Propylenoxid modifizierte Stärke in einer 43 Gew.-%-igen wässrigen Lösung eine Viskosität im Bereich von 150 bis 1500000 mPa·s, bevorzugt von 10000 bis 200000 mPa·s, besonders bevorzugt von 25000 bis 180000 mPa·s, (jeweils Brookfield Viskosimeter, Spindel #7 bei 20°C und 20 U/min) aufweist.

7. Kosmetisches Mittel nach einem der Ansprüche 1 bis 6, **dadurch gekennzeichnet, dass** die nichtionische, mittels Propylenoxid modifizierte Stärke in einer Menge von 0,1 Gew.-% bis 10 Gew.%, besonders bevorzugt von 0,2 Gew.-% bis 5,0 Gew.-%, ganz besonders bevorzugt von 1,0 bis 3,0 Gew.-%, jeweils bezogen auf das Gewicht des Mittels, enthalten ist.

8. Kosmetisches Mittel nach einem der Ansprüche 1 bis 7, **dadurch gekennzeichnet, dass** als nichtionische, mittels Propylenoxid modifizierte Stärke mindestens eine unvernetzte, nichtionische, mittels Propylenoxid modifizierte Stärke enthalten ist.

9. Kosmetisches Mittel nach einem der Ansprüche 1 bis 8, **dadurch gekennzeichnet, dass** es als Schaum vorliegt.

10. Kosmetisches Mittel nach einem der Ansprüche 1 bis 9, **dadurch gekennzeichnet, dass** zusätzlich mindestens ein filmbildendes und/oder festigendes Polymer enthalten ist.

11. Kosmetisches Mittel nach einem der Ansprüche 1 bis 10, **dadurch gekennzeichnet, dass** der kosmetische Träger Wasser enthält, so dass das erfindungsgemäße Mittel mindestens 50 Gew.-% Wasser - bezogen auf das Gewicht des gesamten Mittels - enthält

12. Verwendung eines kosmetischen Mittels nach einem der Ansprüche 1 bis 11 zur temporären Umformung und/oder Formfixierung keratinischer Fasern, insbesondere menschlicher Haare.

13. Verfahren zur temporären Umformung keratinischer Fasern, insbesondere menschlicher Haare, **dadurch gekennzeichnet, dass** ein kosmetisches Mittel nach einem der Ansprüche 1 bis 12 auf die keratinischen Fasern aufgebracht wird.

## Claims

1. A cosmetic agent for temporarily shaping keratin fibers, in particular human hair, containing, in a cosmetic carrier,

   - at least one chitosan, wherein at least one neutralization product of chitosan together with at least one organic carboxylic acid is contained as the chitosan and
   - at least one non-ionic propylene-oxide-modified starch, wherein the non-ionic propylene-oxide-modified starch has an average molecular weight (weight average) of from 700 to 1000 kDa.

2. The cosmetic agent according to claim 1, **characterized in that** the organic carboxylic acid is selected from lactic acid, formic acid, pyrrolidone carboxylic acid, nicotinic acid, hydroxyisobutyric acid, hydroxyisovaleric acid or mixtures thereof.

3. The cosmetic agent according to claim 1 or 2, **characterized in that** the chitosan is contained in a quantity of from 0.01 to 5 wt.%, preferably from 0.05 to 2.0 wt.%, particularly preferably from 0.1 to 1 wt.%, based on the weight of the agent according to the invention in each case.

4. The cosmetic agent according to one of claims 1 to 3, **characterized in that** the non-ionic propylene-oxide-modified starch is a non-ionic propylene-oxide-modified tapioca starch or a non-ionic propylene-oxide-modified potato starch or a mixture of the two aforementioned starches.

5. The cosmetic agent according to one of claims 1 to 4, **characterized in that** the non-ionic propylene-oxide-modified

starch has a propylene oxide content of from 1 to 20 wt.%, preferably from 4 to 12 wt.%, particularly preferably from 9.5 to 10.5 wt.% or from 4.0 to 6.0 wt.%, based on the weight of the modified starch in each case.

6. The cosmetic agent according to one of claims 1 to 5, **characterized in that** the non-ionic propylene-oxide-modified starch has, in a 43 wt.% aqueous solution, a viscosity in the range of from 150 to 1,500,000 mPa·s, preferably from 10,000 to 200,000 mPa·s, particularly preferably from 25,000 to 180,000 mPa·s (in each case Brookfield viscometer, spindle #7 at 20 °C and 20 rpm).

7. The cosmetic agent according to one of claims 1 to 6, **characterized in that** the non-ionic propylene-oxide-modified starch is in a quantity of from 0.1 wt.% to 10 wt.%, particularly preferably from 0.2 wt.% to 5.0 wt.%, more particularly preferably from 1.0 wt.% to 3.0 wt.%, based on the weight of the agent in each case.

8. The cosmetic agent according to one of claims 1 to 7, **characterized in that** at least one non-crosslinked, non-ionic propylene-oxide-modified starch is contained as the non-ionic propylene-oxide-modified starch.

9. The cosmetic agent according to one of claims 1 to 8, **characterized in that** it is in the form of a foam.

10. The cosmetic agent according to one of claims 1 to 9, **characterized in that** it additionally contains at least one film-forming and/or stabilizing polymer.

11. The cosmetic agent according to one of claims 1 to 10, **characterized in that** the cosmetic carrier contains water, such that the agent according to the invention contains at least 50 wt.% water based on the total weight of the agent.

12. The use of a cosmetic agent according to one of claims 1 to 11 for temporarily shaping and/or setting the shape of keratin fibers, in particular human hair.

13. A method for temporarily shaping keratin fibers, in particular human hair, **characterized in that** a cosmetic agent according to one of claims 1 to 12 is applied to the keratin fibers.

**Revendications**

1. Agent cosmétique pour la mise en forme temporaire de fibres kératiniques, en particulier de cheveux humains, contenant dans un support cosmétique

   - au moins un chitosane, au moins un produit de neutralisation de chitosane avec au moins un acide carboxylique organique étant présent en tant que chitosane et
   - au moins un amidon non ionique modifié à l’aide de l’oxyde de propylène, l’amidon non ionique modifié à l’aide de l’oxyde de propylène possédant un poids moléculaire moyen (moyenne en poids) de 700 à 1000 kDa.

2. Agent cosmétique selon la revendication 1 **caractérisé en ce que** l’acide carboxylique organique est sélectionné parmi l’acide lactique, l’acide formique, l’acide pyrrolidone-carboxylique, l’acide nicotinique, l’acide hydroxy-isobutyrique, l’acide hydroxy-isovalérique ou des mélanges de ces acides.

3. Agent cosmétique selon l’une des revendications 1 ou 2 **caractérisé en ce que** le chitosane est contenu dans une quantité de 0,01 % en poids à 5 % en poids, de préférence de 0,05 % en poids à 2,0 % en poids, de manière particulièrement préférée de 0,1 % en poids à 1 % en poids, par rapport au poids de l’agent selon l’invention respectivement.

4. Agent cosmétique selon l’une quelconque des revendications 1 à 3 **caractérisé en ce que** l’amidon non ionique modifié à l’aide de l’oxyde de propylène est un amidon de tapioca non ionique modifié à l’aide de l’oxyde de propylène ou un amidon de pomme de terre non ionique modifié à l’aide de l’oxyde de propylène ou un mélange des deux amidons susmentionnés.

5. Agent cosmétique selon l’une quelconque des revendications 1 à 4 **caractérisé en ce que** l’amidon non ionique modifié à l’aide de l’oxyde de propylène possède une teneur en oxyde de propylène de 1 à 20 % en poids, de préférence une teneur en oxyde de propylène de 4 à 12 % en poids, de manière particulièrement préférée une teneur en oxyde de propylène de 9,5 à 10,5 % en poids ou de 4,0 à 6,0 % en poids, par rapport au poids de l’amidon

modifié respectivement.

6. Agent cosmétique selon l'une quelconque des revendications 1 à 5 **caractérisé en ce que** l'amidon non ionique modifié à l'aide de l'oxyde de propylène possède, dans une solution aqueuse à 43 % en poids, une viscosité comprise dans la plage allant de 150 à 1500000 mPa·s, de préférence de 10000 à 200000 mPa·s, de manière particulièrement préférée de 25000 à 180000 mPa·s (respectivement viscosimètre de Brookfield, broche n°7 à 20 °C et 20 t/min).

7. Agent cosmétique selon l'une des revendications 1 à 6 **caractérisé en ce que** l'amidon non ionique modifié à l'aide de l'oxyde de propylène est présent dans une quantité de 0,1 % en poids à 10 % en poids, de manière particulièrement préférée de 0,2 % en poids à 5,0 % en poids, de manière tout particulièrement préférée de 1,0 à 3,0 % en poids par rapport au poids de l'agent.

8. Agent cosmétique selon l'une quelconque des revendications 1 à 7 **caractérisé en ce qu'**au moins un amidon non ionique modifié à l'aide de l'oxyde de propylène, non réticulé est présent comme amidon non ionique modifié à l'aide de l'oxyde de propylène.

9. Agent cosmétique selon l'une quelconque des revendications 1 à 8 **caractérisé en ce qu'**il se présente sous forme de mousse.

10. Agent cosmétique selon l'une quelconque des revendications 1 à 9 **caractérisé en ce que**, en plus, au moins un polymère filmogène et/ou polymère fixant est présent.

11. Agent cosmétique selon l'une quelconque des revendications 1 à 10 **caractérisé en ce que** le support cosmétique contient de l'eau de telle sorte que l'agent selon l'invention contient au moins 50 % en poids d'eau, par rapport au poids de l'intégralité de l'agent.

12. Utilisation d'un agent cosmétique selon l'une quelconque des revendications 1 à 11 pour la mise en forme temporaire et/ou la fixation de la forme des fibres kératiniques, en particulier de cheveux humains.

13. Procédé de mise en forme temporaire de fibres kératiniques, en particulier de cheveux humains, **caractérisé en ce qu'**un agent cosmétique selon l'une quelconque des revendications 1 à 12 est appliqué sur les fibres kératiniques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE OS19738866 A